# EUROPEAN PATENT SPECIFICATION

(11) **EP 3 969 025 B1**
(45) Date of publication and mention of the grant of the patent: **09.04.2025**
(21) Application number: 20725192.7
(22) Date of filing: 15.05.2020
(51) Int. Cl.: A61K 38/00, A61K 39/00, C07K 5/06

(54) **COMPOSITIONS OF PROTEINS WITH DIPEPTIDES AS STABILISING AGENTS**
ZUSAMMENSETZUNGEN VON PROTEINEN MIT DIPEPTIDEN ALS STABILISIERUNGSMITTELN
COMPOSITIONS DE PROTÉINES AVEC DES DIPEPTIDES COMME AGENTS DE STABILISATION

(30) Priority: 16.05.2019 GB 201906917
(43) Date of publication of application: 23.03.2022
(73) Proprietor: Intract Pharma Limited, London NW1 0NH (GB)
(72) Inventor: BASIT, Abdul, Waseh, London NW1 0NH (GB); YADAV, Vipul, London NW1 0NH (GB)
(74) Representative: Beck Greener LLP
(86) International application number: PCT/EP2020/063728
(87) International publication number: WO 2020/229693

(56) References cited:
- WO-A1-2017/060500
- WO-A1-2017/070501
- WO-A2-2007/122374
- WO-A2-2014/030052
- KENJI SUZUKI: "Amelioration of acute colitis of mice by enema of anti-interferon-inducible-protein 10-antibody", GASTROENTEROLOGY, vol. 126, no. S2, 1 April 2004 (2004-04-01), pages A284, XP055712030
- J. SCOTT CROWE ET AL: "Oral delivery of the anti-tumor necrosis factor [alpha] domain antibody, V565, results in high intestinal and fecal concentrations with minimal systemic exposure in cynomolgus monkeys", DRUG DEVELOPMENT AND INDUSTRIAL PHARMACY, vol. 45, no. 3, 4 March 2019 (2019-03-04), US, pages 387 - 394, XP055711849, ISSN: 0363-9045, DOI: 10.1080/03639045.2018.1542708
- MAURER J M: "ColoPulse tablets in inflammatory bowel disease", INTERNET CITATION, 14 February 2017 (2017-02-14), XP002796749, ISBN: 978-94-6299-525-3, Retrieved from the Internet <URL:https://www.rug.nl/research/portal/files/40323590/Complete_thesis.pdf> [retrieved on 20200108]
- TOZAKI H ET AL: "CHITOSAN CAPSULES FOR COLON-SPECIFIC DRUG DELIVERY: IMPROVEMENT OF INSULIN ABSORPTION FROM THE RAT COLON", JOURNAL OF PHARMACEUTICAL SCIENCES, AMERICAN CHEMICAL SOCIETY AND AMERICAN PHARMACEUTICAL ASSOCIATION, US, vol. 86, no. 9, 1 September 1997 (1997-09-01), pages 1016 - 1021, XP000696914, ISSN: 0022-3549, DOI: 10.1021/JS970018G
- DATABASE BIOSIS [online] BIOSCIENCES INFORMATION SERVICE, PHILADELPHIA, PA, US; 2017, LAZARENKO A A ET AL: "DEVELOPMENT OF THE SUPPOSITORY FORM OF HUMAN IMMUNOGLOBULIN PREPARATION WITH HIGH TITERS OF ANTIBODIES TO HERPES SIMPLEX VIRUS TYPES I AND 2 FOR THE TREATMENT OF CHRONIC FORMS OF HERPETIC DISEASE", XP002799626, Database accession no. PREV201700293618
- VOPROSY VIRUSOLOGII, vol. 62, no. 1, 2017, pages 36 - 41, ISSN: 0507-4088, DOI: 10.18821/0507-4088-2017-62-1-36-41
- HOSNY ET AL: "Relative hypoglycemia of rectal insulin suppositories containing deoxycholic acid, sodium taurocholate, polycarbophil, and their combinations in diabetic rabbits", DRUG DEVELOPMENT AND INDUSTRIAL PHARMACY, NEW YORK, NY, US, vol. 25, no. 6, 1 January 1999 (1999-01-01), pages 745 - 752, XP009164926, ISSN: 0363-9045, DOI: 10.1081/DDC-100102234

## Description

### Field of the Invention

This invention relates to compositions comprising antibodies, which are stabilized in the presence of intestinal fluid by reducing degradation in the ileum and/or colon.

### Background

Biopharmaceuticals, and particularly proteins (including antibodies) and are an increasingly important class of pharmaceuticals, and many therapeutic uses have been proposed and implemented. Monoclonal antibodies (mAbs) are the highest selling class of biotherapeutics. However, delivery of such biopharmaceuticals to the desired target continues to be a major challenge in pharmaceutical industry as well as an unmet medical need for several diseases. Specifically, oral or rectal administration can be ineffective, because the molecules are highly unstable in the presence of gastric and intestinal fluids. Hence for this reason, intravenous or subcutaneous delivery remains the most feasible option for delivery of proteins. However, such delivery is not usually the most convenient method of administration of a drug to a patient, and issues include pain at the site of injection, compliance, and frequent hospital visits for intravenous infusions. There can also be other major disadvantages of intravenous or subcutaneous delivery, and for some applications, high systemic doses of mAbs have been reported to be associated with serious systemic adverse effects. High systemic exposure can also lead to a decline in efficacy with repeated administration due to formation of anti-drug antibodies.

Currently there is no marketed oral biologic therapy available for the treatment of chronic diseases such as diabetes or inflammatory bowel disease (IBD), and there is a distinct unmet medical need for safer, more effective, patient-friendly therapeutic approaches.

For some drugs, the targeting of the drug to the colon has been utilised as a means of achieving local therapy or systemic treatment. For example, WO 2007/122374 describes compositions having a delayed release coating which can be used to target release of a drug from a core to the intestine, particularly the colon. The colon is susceptible to a number of disease states, including IBD, irritable bowel syndrome (IBS), constipation, diarrhoea, infection and carcinoma. IBD is a chronic, medically incurable condition involving inflammation of the gut. There are two main forms of IBD: ulcerative colitis (UC), which typically begins in the descending colon and rectum and may extend continuously to involve the entire colon (pancolitis), and Crohn's disease (CD), which most commonly involves the terminal small intestine and ascending colon. UC usually affects only the epithelial layer of the bowel wall, while CD may affect all layers of the intestinal wall. A number of biological therapies are currently available for the treatment of IBD, but because of the stability problems discussed above, none is currently available as an oral treatment.

Suzuki (2004 Gastroenterology 126(82):A28) describes an enema formulation comprising an antibody, and its use in treating DSS-induced acute colitis (a model of IBD).

Crowe (2019 Drug Devel. Industrial Pharm. 45(3):387-394) and Maurer (2017 PhD Thesis, U. Groningen) describe ways of administering proteins (including an antibody) to the GI tract using capsules. Other suitable types of capsules are described in Tozaki (1997 J. Pharm. Sci 86(9):1016-1021) and WO2007/122374.

Lazarenko (2017 Voprisy Virusologii 62(1):36-41) and Hosny (1999 Drug Dev. Industrial Pharm 25(6):745-752) disclose ways of administering proteins (including antibodies) to the GI tract using suppositories.

The use of camostat mesylate for stabilization of the therapeutic proteins against degradation in the intestinal environment is disclosed in WO 2014/030052.

WO2017/060500 discloses compositions comprising small proteins and carnosine. WO2017/070501 discloses a composition comprising an antibody and a hindered amine like caffeine.

There remains an unmet need for a method of stabilising proteins in the presence of luminal fluid found in the lower gastrointestinal tract, such as the small intestine, and/or the colon. Specifically, there remains a need for a method of stabilising proteins sufficiently for them to be delivered to the ileum and/or the colon by rectal or, especially, oral administration. The present inventors have found that di-peptides (such as for example diglycine and L-carnosine), or combinations of these, are effective in stabilising proteins on contact with body fluids found in the lower gastrointestinal tract, such as the small intestine and/or the colon.

Surprisingly, the present inventors have found that when an enzyme inhibitor is included in compositions containing proteins as active ingredient, together with the dipeptides an enhanced stabilisation in GI fluids is observed. In contrast, the present inventors found that enzyme inhibitors, when used alone, have little effect on the stability of proteins on contact with body fluids found in the ileum and/or the colon.

### Summary of the invention

Accordingly, the present invention provides a pharmaceutical composition which comprises
(i) as active ingredient an antibody or an antigen binding fragment thereof together with
(ii) carnosine and/or diglycine and
(iii) optionally aprotinin, or a fragment, or an analog thereof having at least 70% similarity to SEQ ID NO:1, wherein said analog or fragment of aprotinin acts as an enzyme inhibitor;
and wherein the antibody is stabilized in the presence of intestinal fluid by reducing degradation of the antibody in the ileum and/or colon.

. The carnosine and/or diglycine and aprotinin, or a fragment, or an analog thereof which act as an enzyme inhibitor act as stabilising agents.

In the compositions of the invention, the "active ingredient" is an antibody or an antigen binding fragment thereof.

The composition of the invention may be in liquid or in solid or in semi-solid form, preferably in a form suitable for rectal or, especially, oral administration. The compositions may be in a solid form suitable for oral administration, said composition having an enteric coating. Most preferably it is in a solid or semi-solid form suitable for oral administration, and adapted for selective release of the protein in the lower gastrointestinal tract, in particular the ileum and/or the colon.

The composition may comprise a solid dosage form with a core and a coating for the core. The core may comprise as an active ingredient an antibody or an antigen binding fragment thereof, the di-peptide selected from carnosine and/or diglycine and optionally the enzyme inhibitor selected from aprotinin, or a fragment, or an analog thereof; and the coating may comprise a mixture of a digestible polysaccharide and a film-forming material which has a solubility threshold at pH 6.0 or above.

The composition may be an orally administrable pharmaceutical composition comprising as active ingredient an antibody or an antigen binding fragment thereof, one or more di-peptides selected from carnosine and/or diglycine and optionally an enzyme inhibitor selected from aprotinin, or a fragment, or an analog thereof.

The invention also provides a solid dosage form for oral administration comprising a core comprising as active ingredient an antibody or an antigen binding fragment thereof, one or more di-peptides selected from carnosine and/or diglycine and optionally an enzyme inhibitor selected from aprotinin, or a fragment, or an analog thereof, and a delayed release coating for the core.

The invention also provides a rectally administrable pharmaceutical composition comprising as active ingredient an antibody or an antigen binding fragment thereof, one or more di-peptides selected from carnosine and/or diglycine and optionally an enzyme inhibitor selected from aprotinin, or a fragment, or an analog thereof.

The invention also provides an enema formulation comprising as active ingredient an antibody or an antigen binding fragment thereof, and optionally one or more di-peptides selected from carnosine and/or diglycine and with or without an enzyme inhibitor selected from aprotinin, or a fragment, or an analog thereof.

The present invention further provides a pharmaceutical composition according to the invention for use in therapy. Further, there is also provided a pharmaceutical composition according to the invention for use in the treatment or prevention of disease or conditions selected from an inflammatory bowel disease; irritable bowel syndrome; constipation; diarrhoea; infection; or cancer. Preferably the pharmaceutical composition is a pharmaceutical composition of the invention.

The invention further provides a method of treating or preventing a disease or condition in a subject which comprises administering to the subject a pharmaceutical composition according to the invention. Preferably, the disease or condition is selected from inflammatory bowel disease; irritable bowel syndrome; constipation; diarrhoea; infection; autoimmune disease or cancer.

The present invention further provides a method of stabilising an antibody or an antigen binding fragment thereof in the presence of intestinal fluid, which comprises contacting the antibody or an antigen binding fragment thereof with one or more di-peptides selected from carnosine and/or diglycine and optionally an enzyme inhibitor selected from aprotinin, or a fragment, or an analog thereof.

The invention also provides the use of one or more di-peptides selected from carnosine and/or diglycine and optionally an enzyme inhibitor selected from aprotinin, or a fragment, or an analog thereof for the stabilisation of an antibody or an antigen binding fragment thereof which has been administered as a pharmaceutical composition and delivered to the lower gastro-intestinal tract. Preferably the pharmaceutical composition is a pharmaceutical composition of the invention.

Following release, penetration of the drug into GI tissue at or close to the site of release in the GI lumen can occur, allowing for local treatment of diseases of the GI tract, and, through penetration of the drug into the bloodstream, treatment of a large range of diseases.

### Detailed description of the invention

As used herein "lower gastrointestinal tract" refers to gastrointestinal tract after the stomach. This includes the small intestine and large intestine. The small intestine is made up of the duodenum, jejunum and ileum, while the large intestine is also known as the colon. The one or more di-peptides selected from carnosine and/or diglycine are used as stabilising agents to reduce degradation of the protein in the ileum and/or colon.

### Protein therapeutics

The active ingredient may be any antibody or an antigen binding fragment thereof whose therapeutic effect is advantageously realised by administration via the lower gastrointestinal tract, preferably to the ileum and/or the colon, especially the colon.

### Antibodies

The active ingredient is one or more antibodies.

As used herein, "antibody" means an immunoglobulin molecule that recognizes and specifically binds to a target antigen, such as a cytokine, protein, polypeptide, peptide, carbohydrate, polynucleotide, lipid, bacteria or virus, or combination thereof through at least one antigen recognition site within the variable region of the immunoglobulin molecule. The term "antibody" encompasses polyclonal antibodies, monoclonal antibodies, multispecific antibodies such as bispecific antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. Preferably the antibody is a monoclonal antibody. An antibody can include any of the five major classes of immunoglobulins: IgA, IgD, IgE, IgG, and IgM, or subclasses (isotypes) thereof (e.g. IgGI, IgG2, IgG3, IgG4, IgAl and IgA2), based on the identity of their heavy-chain constant domains referred to as alpha, delta, epsilon, gamma, and mu, respectively. The different classes of immunoglobulins have different and well known subunit structures and three-dimensional configurations. Preferably, the antibody is an IgG antibody, more preferably an IgGI or IgG4. The term "antibody" is also intended to include conjugates of the antibody, for example conjugates with polyethylene glycol, PEG.

Further, except where the context requires otherwise, the term "antibody" should be understood to encompass complete antibodies and antibody fragments comprising an antigen-binding region of the complete antibody. Antibody fragments may for example be single domain antibodies (e.g. V_{H}H domain antibodies) monovalent or divalent Fab, Fab', F(ab')2, scFv, Fc, bispecific antibodies, diabodies, minibodies or multispecific antibodies formed from antibody fragments, for example minibodies composed of different permutations of scFv fragments or diabodies, and optionally Fc fragments or CH domains, such as scFv-Fc, scFv-Fc-scFv, Fab-scFv, (Fab'ScFv)2, scDiabodies, scDiabody-Fc, scDiabody-CH3, scFv-CH3, and scFv-CH2-CH3 fusion proteins. An antibody fragment can be produced by enzymatic cleavage of a complete antibody, or by synthetic means such as recombinant DNA techniques, phage display or yeast display technologies or using transgenic mice, or liquid or solid phase peptide synthesis. Antibody fragments may also form part of a fusion protein, provided that the antigen- binding ability is retained.

Suitable antibodies for use in the invention include adalimumab, infliximab, ruplizumab, cetrolizumab pegol, golimumab, natalizumab, vedolizumab, tildrakizumab, ustekinumab and combinations thereof.

Where an antibody is used in a composition according to the invention, it may be any one whose therapeutic effect is advantageously realised by administration via the colon. Specific antibodies of particular interest in the context of the present invention include existing commercial IBD therapeutic antibodies such as adalimumab, infliximab, cetrolizumab pegol, golimumab, natalizumab, vedolizumab, tildrakizumab, ustekinumab, and additional antibodies in development for IBD treatment which target pathways and molecules (agonists or antagonists) implicated in pathogenesis of IBD, such as, for example CD40. Targeting to the colon additionally affords the possibility to improve treatment of colorectal cancer by targeting and localization of anti-cancer therapeutic antibodies, or of different possible formats as mentioned above, to the tumour.

Further, it is recognised that stabilisation of biomolecules in the gastrointestinal (GI) tract and penetration into GI tissues additionally may offer the potential for transmission of therapeutics through GI tissue and into the systemic circulation, thus affording the opportunity to target a much larger range of diseases.

### Stabilising agents

The di-peptides and enzyme inhibitor are used as stabilising agents.

The stabilising agents help to maintain the intact protein in the lower gastrointestinal tract. Methods for assessing the stability of the antibody in gastrointestinal fluid are described in the Examples. The one or more di-peptides and optionally the enzyme inhibitor cause at least 5% of the antibody present to remain intact after 4 hours in the gastrointestinal fluid. Preferably at least 10%, 20%, 30%, 40%, 50%, 60%, 70%, 75%, 80%, 90% 95% or more of the antibody remains intact.

### Dipeptides

The compositions of the invention contain at least one, preferably at least two dipeptides, which are diglycine and/or L-carnosine.

Dipeptides are molecules comprising 2 amino-acids linked by a peptide bond. The amino acids can be naturally occurring or chemically modified versions. Examples of di-peptides are diglycine or L-carnosine, as discussed below.

### Diglycine

Diglycine is a dipeptide which consists of two glycine amino acids linked together through a peptide bond. It has the following structure.

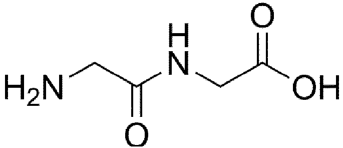

### L-Carnosine

L-Carnosine (also known as beta-alanyl-L-histidine) is a dipeptide of beta-alanine and histidine which has the following structure.

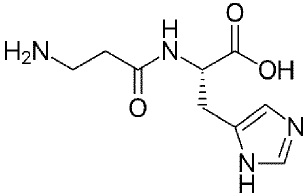

### Enzyme inhibitors

Preferably the composition comprises an enzyme inhibitor selected from aprotinin, or a fragment, or an analog thereof as a further stabilising agent.

Preferably, the enzyme inhibitor is aprotinin and the di-peptide is di-glycine and/or L-carnosine. The composition may comprise aprotinin, di-glycine and L-carnosine as stabilising agents.

### Aprotinin

The pharmaceutical composition of the invention may comprise aprotinin, an analogue of aprotinin or a fragment thereof. It will of course be understood that aprotinin, analogues of aprotinin or fragments thereof are not present in the composition of the invention as an active ingredient.

Aprotinin is a protease inhibitor, which is known to inhibit trypsin and other similar proteases. It is often also referred to as Bovine Pancreatic Trypsin Inhibitor (BPTI). Aprotinin is a 58 amino acid protein that is formed after processing of a 100 amino acid polypeptide that comprises a signal peptide, propeptide domains, and a Kunitz domain. Aprotinin in its mature 58 amino acid form is available commercially and is also sold under the trade name Trasylol^{®}, which is indicated for prophylactic use to reduce blood loss during surgery.

The aprotinin for use in the invention may comprise the amino acid sequence
RPDFCLEPPYTGPCKARMIRYFYNAKAGLCQPFVYGGCRAKRNNFKSSEDCMRTCGGA (SEQ ID NO 1).

The aprotinin or fragment thereof, or aprotinin analogue or fragment thereof for use in the invention may be prepared recombinantly (for example, in *E. coli,* mammalian cells or insect cells), synthetically (for example, using standard organic chemistry techniques, such as solution or solid phase peptide synthesis), or it may be a native protein from an animal source, such as a bovine source.

Further, except where the context requires otherwise, the term "aprotinin" should be understood to encompass aprotinin, analogues of aprotinin, fragments of aprotinin and fragments of analogues of aprotinin. Suitable analogues and fragments for use in the present invention are those which when combined with one or more dipeptides enhance the stability of a protein in the presence of the body fluids found in the lower gastrointestinal tract, for example the ileum and/or the colon. Methods for confirming the ability of aprotinin, a fragment of aprotinin, an analogue of aprotinin or a fragment thereof to enhance the stability of the active ingredient (an antibody) in the presence of the body fluids found in the lower gastrointestinal tract, such as the ileum and/or the colon are described in the Examples below. "Enhanced" stability as used herein means that at least 50% of the active ingredient present remains intact after 4 hours in the gastrointestinal fluid. Preferably at least 60%, 70%, 75%, 80%, 90% 95% or more of the active ingredient remains intact.

For example, aprotinin fragments for use in the present invention may comprise a fragment of the sequence defined by SEQ ID NO 1. For example, the aprotinin fragment may comprise at least 30, 40, 50, 51, 52, 53, 54, 55, 56 or 57 contiguous amino acids of the sequence defined by SEQ ID NO 1.

Analogues of aprotinin for use in the present invention may include proteins comprising a sequence similar to the amino acid sequence defined in SEQ ID NO 1, and which when combined with one or more dipeptides enhance the stability of an antibody in the presence of the body fluids found in the lower gastrointestinal tract, such as the ileum and/or the colon. For example, the aprotinin analogue may have at least 70%, 80%, 90% or at least 95% similarity to the sequence defined in SEQ ID NO 1 or a fragment of the sequence defined by SEQ ID NO 1. Alternatively, the aprotinin analogue may have at least 70%, 80%, 90% or at least 95% identity to the sequence defined in SEQ ID NO 1 or a fragment of the sequence defined by SEQ ID NO 1. For example, the aprotinin analogue may comprise an amino acid sequence that is identical to 50, 51, 52, 53, 54, 55, 56 or 57 amino acids of the sequence defined by SEQ ID NO 1. The similar or identical amino acids may be contiguous or non-contiguous.

A program such as the CLUSTAL program to can be used to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention. Identity or similarity is preferably calculated over the entire length of SEQ. ID No:1.

The analogue of aprotinin may contain one or more amino acid substitutions, insertions and/or deletions.

Amino acid substitution means that an amino acid residue is substituted for a replacement amino acid residue at the same position. Amino acid substitutions may be conservative, by which it is meant the substituted amino acid has similar chemical properties to the original amino acid. A skilled person would understand which amino acids share similar chemical properties. For example, the following groups of amino acids share similar chemical properties such as size, charge and polarity: Group 1 Ala, Ser, Thr, Pro, Gly; Group 2 Asp, Asn, Glu, Gln; Group 3 His, Arg, Lys; Group 4 Met, Leu, Ile, Val, Cys; Group 5 Phe Thy Trp.

Inserted amino acid residues may be inserted at any position and may be inserted such that some or all of the inserted amino acid residues are immediately adjacent one another or may be inserted such that none of the inserted amino acid residues is immediately adjacent another inserted amino acid residue. For example, the aprotinin analogue may comprise 1, 2, 3, 4, 5, 6, 7, 8, 9 or 10 additional amino acids at the N- and/or C-terminus of the amino acid sequence defined in SEQ ID NO 1.

One, two or three amino acids may be deleted from the sequence of SEQ ID NO: 1. Each deletion can take place at any position of SEQ ID NO: 1.

Inserted amino acids and replacement amino acids may be naturally occurring amino acids or may be non-naturally occurring amino acids and, for example, may contain a non-natural side chain, and/or be linked together via non-native peptide bonds. Such altered peptide ligands are known in the art. If more than one amino acid residue is substituted and/or inserted, the replacement/inserted amino acid residues may be the same as each other or different from one another. Each replacement amino acid may have a different side chain to the amino acid being replaced. Analogues of aprotinin may comprises one or more modified bases, wherein the amino acid residues may be chemically modified. Examples of chemical modifications include those corresponding to post translational modifications for example phosphorylation, acetylation and deamidation. Chemical modifications may not correspond to those that may be present in vivo. For example, the N or C terminal ends of the peptide may be modified improve the stability, bioavailability and or affinity of the peptides. Further examples of non-natural modifications include incorporation of non-encoded α-amino acids, photoreactive cross-linking amino acids, N-methylated amino acids, and β-amino acids, backbone reduction, retroinversion by using d-amino acids, N-terminal methylation and C-terminal amidation and pegylation.

### Pharmaceutical formulations

The pharmaceutical compositions according to the invention are preferably in liquid, solid or semi-solid form, and preferably they are suitable for oral or rectal administration.

The composition may also be in the form of a lotion, cream, foam, emulsion or gel. Such formulations may be prepared by a number of known methods established in the art.

For example, the active ingredient (antibody) and the required stabilising agent may be admixed together, optionally together with other excipients required in the dosage form. Pharmaceutical compositions in the present invention that are suitable for oral administration may be presented either in the form of tablets, capsules, mini-tablets, pellets, powders, granules, microparticles, nanoparticles or hydrogels.

Compositions of the present invention suitable for oral administration may be presented as discrete units such as capsules, tablets, mini-tablets, or pellets, or as powders, granules or crystals. In a solid composition, the minimum diameter of each particle is typically at least 10⁻⁴m, usually at least 5 x 10⁻⁴m and, preferably at least 10⁻³m. The maximum diameter is usually no more than 30 mm, typically no more than 20 mm and, preferably, no more than 10 mm. In preferred embodiments, the particle has a diameter from about 0.2 mm to about 15 mm, preferably from about 1 mm to about 4 mm (e.g. for pellets or mini-tablets) or from about 6 mm to about 12 mm (e.g. for certain tablets or capsules). The term "diameter" refers to the largest linear dimension through the particle.

As well as the required stabilising agents, compositions according to the invention may of course contain any further conventional excipients as required such as binders, extenders, disintegrants, diluents and lubricants. Excipients used in solid forms include for example, microcrystalline cellulose, dicalcium phosphate, starch, magnesium stearate, calcium sulfate, sorbitol, glucose and/or lactose and/or other excipients, binders, extenders, disintegrants, diluents and lubricants known in the art. Suitable binders include starch, gelatine, natural sugars such as glucose or beta-lactose, corn sweeteners, natural and synthetic gums such as acacia, tragacanth or sodium alginate, carboxymethylcellulose, polyethylene glycol, waxes and the like. Disintegrators include without limitation starch, methylcellulose, agar, bentonite, xanthan gum and the like. Fast dissolving diluents include mannitol, lactose, sucrose and/or cyclodextrins. Lubricants, glidants, flavours, colouring agents and stabilizers may also be added for ease of fabrication and use. Lubricants include sodium oleate, sodium stearate, magnesium stearate, sodium benzoate, sodium acetate, sodium chloride.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow, delayed or controlled release of the antibody. Preferred examples of coatings are given below.

Capsules may have solid, semi-solid or non-solid contents. Exemplary contents for capsules may include suspensions which can contain, for example, microcrystalline cellulose for imparting bulk, alginic acid or sodium alginate as a suspending agent, and methylcellulose as a viscosity enhancer, as well as any of the solid or semi-solid forms above.

Formulations for rectal administration may be presented as a suppository with the usual carriers such as cocoa butter, synthetic glyceride esters or polyethylene glycol. Such carriers are typically solid at ordinary room temperatures (upto 25°C), but liquefy and/or dissolve in the rectal cavity to release the drug.

Compositions may also take the form of an enema formulation such as a liquid or foam enema which is rectally administered to the lower colon. The enema formulations typically comprise the protein together with the stabilising agents dissolved or dispersed in a suitable flowable carrier vehicle, such as deionised and/or distilled water. The formulation can be thickened with one or more thickeners. They may also contain a buffer and can also comprise an effective amount of a lubricant such as a natural or synthetic fat or oil, e.g. a tris-fatty acid glycerate or lecithin. Non-toxic non-ionic surfactants can also be included as wetting agents and dispersants. A buffer is preferably added to the liquid or foam enema to stabilise the pH. The pH is preferably 3.5 to 7.5, especially 6.5 to 7.5.

Unit doses of enema formulations can be administered from pre-filled bags or syringes. In the case of a pressurised enema formulation the carrier vehicle may also comprise an effective amount of a foaming agent such as n-butane, propane or i-butane, or the foaming agent/propellant could be held separately from the composition such as in a bag-in-bag or bag-in-can system as described in WO-A-9603115 (incorporated herein by reference). Enema foams may also comprise expanding agents and foam-stabilisers.

The volume of a liquid enema is typically 50-200 cm³, preferably about 100 cm³. The volume of a foam enema is typically 20 to 40cm³. A suitable dosage of the stabilising agent, in particularly aprotinin, in the enema as administered is 1mg/ml to 20mg/ml, preferably 2mg/ml to 10mg/ml. The di-peptide is typically present in an amount resulting in a concentration of 0.1-100mM, typically 25-80 mM, or 50-75mM.

Preferred unit dosage formulations are those containing an effective dose, or an appropriate fraction thereof, of the active ingredient. Release from certain formulations may also be sustained, if the composition contains suitable controlled-release excipients. However, in preferred formulations, release is pulsatile.

The compositions according to the invention will typically comprise a therapeutically effective amount of the active ingredient (antibody) which may be from 0.01 wt % to 99 wt %, based on the total weight of the composition. The actual dosage would be determined by the skilled person using common general knowledge. However, by way of example, "low" dose formulations typically comprise no more than 20 wt % of the active ingredient, and preferably comprise from 1 wt % to 10 wt %, e.g. 5 wt %, of the active ingredient. "High" dose formulations typically comprise at least 40 wt % of the active ingredient, and preferably from 45 wt % to about 85 wt %, e.g. 50 wt % or 80 wt %.

The compositions according to the invention will typically comprise an effective amount of the stabilising agents. Typically, formulations comprise 0.01 wt % to 99 wt % of the stabilising agents, based on the total weight of the composition. The compositions may preferably comprise no more than 20 wt % of the stabilising agents, and preferably comprise from 1 wt % to 10 wt %, e.g. 5 wt %, of the stabilising agents. Alternatively the compositions may comprise at least 40 wt % of the stabilising agents, and preferably from 45 wt % to about 85 wt %, e.g. 50 wt % or 80 wt %.

The di-peptide is typically present in an amount resulting in a concentration of 0.1-100mM, typically 25-80 mM, or 50-75mM in the lower gastrointestinal tract. Each dosage form may contain 50-5000mg di-peptide, typically 100-1000mg; 250-750mg or 300-500mg.

Aprotinin is typically present in an amount resulting in a concentration of 0.1-20 mg/ml in the lower gastrointestinal tract. Each dosage form may contain 50-5000mg aprotinin, typically 100-1000mg; 250-750mg or 300-500mg.

Whilst the antibody may be used as the sole active ingredient in a composition according to the invention, it is also possible for the antibody to be used in combination with one or more further therapeutic agents. Thus, the invention also provides a composition according to the invention containing a further therapeutic agent in addition to the antibody. If desired, the composition according to the invention may be administered together with a further composition, by simultaneous, sequential or separate administration.

Except where the context requires otherwise, throughout this Specification and claims, any reference to a pharmaceutical composition in solid or semi-solid form should be understood to include individual solid or semi-solid particles or unit forms which are solid or semi-solid throughout, as well as those having a solid or semi-solid exterior and a non-solid, for example liquid or gel, interior. For example, a capsule may have liquid or gel contents.

### Delivery to the lower gastrointestinal tract

The composition according to the invention is adapted for delayed or selective release of the active ingredient in the lower gastrointestinal tract, in particular the ileum and/or, especially, the colon, suitably following rectal or, especially, oral administration. This may be accomplished by the use of particular coatings. The compositions of the invention may be delayed release oral (DRO) compositions. The DRO compositions pass through the stomach substantially unaltered and deliver the active ingredient to the lower gastrointestinal tract, typically the ileum and/or colon (i.e. the site of the diseased mucosa).

The compositions according to the invention may have an enteric coating. Enteric coatings protect the active ingredients in a composition from attack and degradation in the stomach, but dissolve and release to contents of the dosage form within the intestines, usually due to the change in pH. Suitable enteric coatings are well known in the art. The optimal coating for any particular formulation depends on the exact intended use, and coatings may be tailored to release the active ingredient in a particular region of the intestines, or at a particular time following ingestion. Such a formulation may if desired contain one or more intermediate layers between the active ingredient and the outer enteric coating. In this case, it is possible for a composition of the invention to release a portion of its contents at one particular region of the intestine, and a further portion of its contents in a second region of the intestine, such as the colon. Preferably, the composition of the present invention is in a solid or semi-solid form which comprises an enteric coating adapted to release the protein in the colon. Useful enteric coatings are those which remain intact in the low pH environment of the stomach, but readily dissolve when the optimum pH for dissolution is reached. This can vary between pH 3 to 7.5, preferably 5 to 7, depending on the chemical composition of the coating. The thickness of the coating required will depend on the solubility of the coating and the intended site to be treated. Typically the coating is 25 to 200µm, especially 75 to 150 µm.

The composition of the invention is adapted for release of the active ingredient to the part of the lower gastrointestinal tract where the disease is prevalent. Typically the enteric coating should dissolve in the pH of the jejunum (about pH 5.5), ileum (about pH 6) and/or colon (pH 6-7) to that the majority of the therapeutic molecule is released at the desired site. WO 2007/122374 (the contents of which are incorporated herein by reference) describes compositions for selective release within the colon, and these form one preferred embodiment of the invention. Accordingly, the invention further provides a composition comprising a particle with a core and a coating for the core, the core comprising the active ingredient together with one or more di-peptides and optionally an enzyme inhibitor, the coating comprising a mixture of a mixture of a digestible polysaccharide and a film-forming material which has a solubility threshold at pH 6.0 or above, preferably pH 7 or above.

The digestible polysaccharide is susceptible to attack by intestinal bacteria. Preferably the digestible polysaccharide. The digestible polysaccharide is preferably selected from the group consisting of starch; amylose; amylopectin; chitosan; chondroitin sulfate; cyclodextrin; dextran; pullulan; carrageenan; scleroglucan; chitin; curdulan and levan.

For example, the polysaccharide may be starch, amylose or amylopectin.

The film-forming material is an enteric material which has a pH threshold which is the pH below which it is insoluble and at or above which it is soluble. The pH of the surrounding medium triggers dissolution of the second material. The normal pH of gastric juice is usually in the range of 1 to 3, while the pH of intestinal juice gradually increases from about 5.5 in the duodenum to about 7 to 8 in the colon. Thus, the second material, when used in a composition of the present invention, has a pH threshold of 6.0 or greater, especially 7 or greater.

The film-forming material is typically selected from an acrylate polymer, a cellulose polymer or a polyvinyl-based polymer. Examples of suitable cellulose polymers include cellulose acetate phthalate ("CAP"); cellulose acetate trimellitate ("CAT"); and hydropropylmethylcellulose acetate succinate. Examples of suitable polyvinyl-based polymers include polyvinyl acetate phthalate ("PVAP"). The film-forming material is preferably a co-polymer of a (meth)acrylic acid and a (meth)acrylic acid C_{I-4} alkyl ester, for instance, a copolymer of methacrylic acid and methacrylic acid methyl ester. Such polymers include those available under the Trade Marks Eudragit L, Eudragit S and Eudragit FS. The use of Eudragit S as the film-forming material is particularly preferred.

In such compositions, multi-unit dosage forms comprising particles having a diameter of less than 3mm are preferred. The "core" is usually a single solid body. The core may consist of the active ingredient (protein ) together with one or more di-peptides and optionally an enzyme inhibitor. More usually, however, the core will comprise a mixture of the active ingredient and the stabilising agents, and optionally one or more additional excipient. The core may for example include a filler or diluent material, e.g. lactose or cellulose material such as microcrystalline cellulose; a binder, e.g. polyvinylpyrrolidone (PVP); a disintegrant, e.g. croscarmellose sodium; and/or a lubricant, e.g. magnesium stearate. The core may be a compressed granulate comprising at least some of these materials.

Release from such compositions is delayed until the lower gastro-intestinal tract, in particular the ileum and/or the colon. Such compositions have application in a multi-phasic release composition comprising at least two pluralities of particles, e.g. coated pellets, in the same dosage form, e.g. a capsule, in which the particles of one plurality are differentiated from the particles of the or each other plurality by the coating. The coatings may differ from one plurality to the next in terms of coating thickness or composition, e.g. the ratio and/or identity of components. Multi-phasic release formulations would be particularly suitable for suffers of Crohn's disease affecting different regions along the intestine, including the ileum and/or the colon.

### Medical applications

The present invention provides a pharmaceutical composition according to the invention for use in therapy. It also provides a method of treating or preventing a disease or condition in a subject, especially a human subject, which comprises administering to the subject via the lower gastro-intestinal tract, especially the ileum and/or the colon a pharmaceutical composition which comprises as active ingredient antibody, together with one or more di-peptides selected from carnosine and/or diglycine and optionally an enzyme inhibitor selected from aprotinin, or a fragment, or an analog thereof. Preferably, the compositions are adapted for administration via the oral or rectal route. Although the invention finds utility in the treatment of diseases of the lower gastro-intestinal tract, especially the ileum and/or the colon, it also has application as a portal for entry of antibody into the systemic circulation by absorption from the lower gastro-intestinal tract, especially the ileum and/or the colon, and hence finds utility in the treatment of a wide range of diseases and conditions. It may for example find utility in the treatment or prevention of autoimmune diseases.

The invention finds particular utility in the treatment or prevention, including maintenance of remission or prevention of relapse, of a disease or condition of the ileum and/or the colon, especially the colon, for example inflammatory bowel disease (including ulcerative colitis and Crohn's disease), IBS, constipation, diarrhoea, infections, or cancer, and the invention therefore further provides the use of one or more di-peptides optionally together with an enzyme inhibitor, in a method of manufacture of a medicament comprising antibody for the treatment and/or prevention of one or more of these conditions. The treatment and/or prevention of IBD is of particular importance.

The following Examples illustrate the invention which refer to the following figures.

Figure 1 shows the level of the antibody in tissues following administration via an enteric coated capsule with and without the stabilising agents, or intravenously.

Figure 2 shows the plasma level of the antibody following administration via an enteric coated capsule with and without the stabilising agents, or intravenously.

### Materials and methods

### Human Colon Model

A human colonic model based on a mixed faecal inoculum was used to mimic the luminal environment of the human large intestine. An anaerobic workstation (Electrotek 500TG^{™} workstation, Electrotek, West Yorkshire, UK) maintained at 37°C and 70% relative air humidity was used to set up the model. The fecal material was transferred in the anaerobic workstation and diluted with freshly prepared basal medium to obtain 20% w/w slurry by homogenization. The basal media provides nutrients and growth factors to the microbiota allowing viability for up to 24 hours. The homogenized bacterial media was sieved through an open mesh fabric (SefarNitexTM, pore size 350 µm) to remove any nonhomogeneous fibrous material. The pH was maintained at approximately 7 to mimic the colonic luminal pH of the human.

### Antibody incubation studies

Antibody stock solution (vedolizumab, ruplizumab, infliximab, ustekinumab, infliximab Fab fragment or combination of infliximab and ustekinumab) was prepared in PBS at 2 mg/ml and added to 20% human or rat faecal slurry to obtain an incubation concentration of 1 mg/ml and 10% w/w faecal slurry. Samples were withdrawn at appropriate time points and added to a protease inhibitor cocktail (Sigma-Aldrich, P2714) in a ratio of 1:3. The samples were centrifuged at 9.6 g for 10 mins and the supernatant was analysed by size exclusion-HPLC (SE-HPLC).

### SE-HPLC

Sample analysis was performed using a high performance liquid chromatography (HPLC) system (Agilent Technologies, 1260 Infinity II Series ^{™}) equipped with a pump (model G1311C), autosampler (model G1329B) and a diode-array UV detector (model G1314B). A 600 × 7.8-mm Biosep^{™} 5 µm SEC-s3000 290 Å (Phenomenex, Torrance, CA) size exclusion (SE) chromatography column was used for sample separation using phosphate buffer saline (pH 7.3) prepared in sterile HPLC grade water as the mobile phase for elution, at a flow rate of 1 ml/min. The analysis was operated at room temperature and UV detection wavelength was set at 280 nm. Each sample was run for 40 minutes to allow complete elution of the sample proteins and reduce run-over. The retention time for IgG1 antibody, F(ab')₂ and Fab/Fc fragments was 17, 18.2 and 20.3 minutes, respectively.

### In-vivo dosing of infliximab capsules with and without stabilizing agents to Wistar Rats

Lyophilized antibody (Infliximab) was formulated into rat capsules (5mm x 2.6mm) at a dose of 0.7mg/kg co-formulated with mannitol or stabilizing agents (aprotinin, L-carnosine and diglycine combination). The capsules were coated with an enteric coating that consisted of a mixture of pH sensitive polymer and intestinal bacteria digestible polysaccharide.

Healthy male Wistar rats (Charles River Laboratories) weighing 350-400g were housed in groups of 4 per cage and acclimatized for at least 5 days prior to entering the study. The rats were dosed with the capsules after overnight fasting and post dosing until sacrificed. The animals had free access to water throughout the study. The rats were sacrificed after 7hrs post dosing and the entire GI tract, plasma and feces were collected, and antibody levels were analyzed by ELISA.

A parallel arm involving IV injection of the same dose of antibody was also tested in rats to compare the tissue and plasma concentration of antibody.

### ELISA

Each well of the 96 well plate was coated with 100 µL of 1 µg/mL antigen (recombinant Human TNF-α Protein) diluted in PBS and incubated overnight at 4°C. The wells were washed 3 times with 200 µL/well washing buffer and blocked for 1hr at 37 °C. Following washing, the test samples were added and incubated for 1hr at 37 °C. After three washing cycles, the secondary antibody solution was added (goat anti-human IgG Fc secondary antibody HRP-conjugated) and incubated for 45 min at 37 °C. After washing steps, the wells were incubated with 150 µL/well of K-BLUE substrate and incubate for 10 min at room temperature in the dark. After 10 minutes, 50 µL of RED STOP solution was added to each well and the plate was read at 650 nm.

### Example 1 Stability of monoclonal antibody in the Human Colon model in the presence of L-carnosine, diglycine (Gly-Gly) and optionally aprotinin

Colon stability was assessed using the Human Colon model with the amount of intact antibody (vedolizumab, ruplizumab, infliximab or ustekinumab) remaining at each time point assessed by SE-HPLC as described in the Methods section. The experiment was carried out in the absence of a dipeptide, aprotinin or an excipient, and then in the presence of 50mM or 100mM L-carnosine. The experiment was also carried out in the presence of L-carnosine together with 0.5 mg/ml aprotinin (Sigma-Aldrich).

The results are shown in the following Table.

| Formulation | Concentration of excipient | % Antibody remaining after 0 hours (T= 0 h) | % Antibody remaining after 4 hours (T= 4 h) | % Antibody remaining after 6 hours (T= 6 h) |
|---|---|---|---|---|
| Infliximab alone | 1 mg/ml | 100 | 0 | 0 |
| Ustekinumab alone | 1 mg/ml | 100 | 2.12 ± 0.20 | 0 |
| Infliximab + L-carnosine | 10mM | 100 | 1.1 ± 0.02 | 0 |
| Infliximab + L-carnosine | 50mM | 100 | 6.27 ± 0.55 | 1.76 ± 0.31 |
| Infliximab + L-carnosine | 100mM | 100 | 17.57 ± 1.23 | 7.84 ± 0.74 |
| Infliximab + diglycine | 100 mM | 100 | 23.25 ± 0.82 | - |
| Infliximab + aprotinin | 0.5 mg/ml | 100 | 20.2 ± 0.16 | 0 |
| Infliximab + aprotinin | 0.1 mg/ml | 100 | 3.05 | - |
| Infliximab + aprotinin + L-carnosine | 0.1 mg/ml + 50 mM | 100 | 73.4 ± 0.05 | - |
| Infliximab + aprotinin + diglycine | 0.1 mg/ml + 50 mM | 100 | 66.24 ± 1.09 | - |
| Infliximab + aprotinin + L-carnosine + diglycine | 0.1 mg/ml + 50 mM + 50 mM | 100 | 76.86 ± 1.39 | - |
| Infliximab + aprotinin + L-carnosine + diglycine | 0.5 mg/ml + 50 mM + 50 mM | 100 | 81.60 ± 3.74 | - |
| Infliximab + aprotinin + L-carnosine + diglycine | 0.5 mg/ml + 25 mM + 50 mM | 100 | 60.69 ± 3.61 | 35.57 ± 2.15 |
| Infliximab + aprotinin + L-carnosine + diglycine | 0.5 mg/ml + 10 mM + 50 mM | 100 | 55.82 ± 2.86 | 27.35 ± 1.76 |
| Ustekinumab + aprotinin + L-carnosine + diglycine | 0.5 mg/ml + 50 mM + 50 mM | 100 | 41.21 ± 1.94 | 14.19 ± 1.11 |

The results show that infliximab, vedolizumab and ruplizumab were degraded by the human colonic microbiota in the absence of the tested stabilizer molecules. The antibody + aprotinin (0.5 mg/ml) control was observed to result in less degradation of the antibody. In the presence of 100 mM L-carnosine and 100 mM diglycine, a significant stabilising effect of the antibodies was obtained. This stabilisation effect of L-carnosine was significantly enhanced by including 0.1 and 0.5 mg/mL aprotinin in the test sample.

### Example 2 Stability of combination of monoclonal antibodies infliximab and ustekinumab in the Human Colon model in the presence of aprotinin, L-carnosine and diglycine (Gly-Gly)

Colon stability was assessed using the Human Colon model with the amount of intact antibody (infliximab, ustekinumab or combination of infliximab and ustekinumab) remaining at each time point assessed by SE-HPLC as described in the Methods section. The experiment was carried out in the absence of a dipeptide, aprotinin or an excipient, and then in the presence of L-carnosine, diglycine and aprotinin (Sigma-Aldrich).

The results are shown in the following Table.

| Formulation | Concentration of excipient | % Antibody remaining after 0 hours (T= 0 h) | % Antibody remaining after 4 hours (T= 4 h) | % Antibody remaining after 6 hours (T= 6 h) |
|---|---|---|---|---|
| Infliximab alone | 1 mg/ml | 100 | 12.34 ± 0.83 | 2.45 ± 0.66 |
| Ustekinumab alone | 1 mg/ml | 100 | 2.12 ± 0.20 | 0 |
| Infliximab + Ustekinumab | 0.5 mg/ml + 0.5 mg/ml | 100 | 6.48 ± 1.01 | 0 |
| Infliximab + aprotinin + L-carnosine + diglycine | 0.5 mg/ml + 50 mM + 50 mM | 100 | 81.60 ± 3.74 | - |
| Ustekinumab + aprotinin + L-carnosine + diglycine | 0.5 mg/ml + 50 mM + 50 mM | 100 | 41.21 ± 1.94 | 14.19 ± 1.11 |
| Infliximab + Ustekinumab + aprotinin + L-carnosine + diglycine | 0.5 mg/ml + 50 mM + 50 mM | 100 | 42.43 ± 1.99 | 17.56 ± 0.44 |

### Example 3 In-vivo PK of antibody in Wistar rats after oral dosing in capsule

Intestinal tissue concentration of antibody (infliximab) was measured after oral capsule dosing with and without stabilization agents (aprotinin, L-carnosine and diglycine combination) and post IV injection at a dose of 0.7 mg/kg.

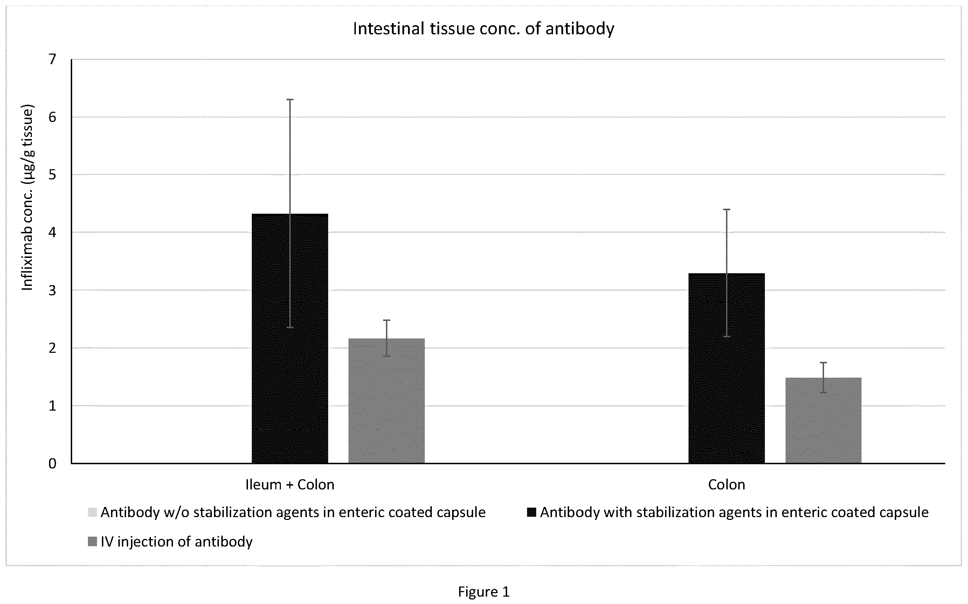

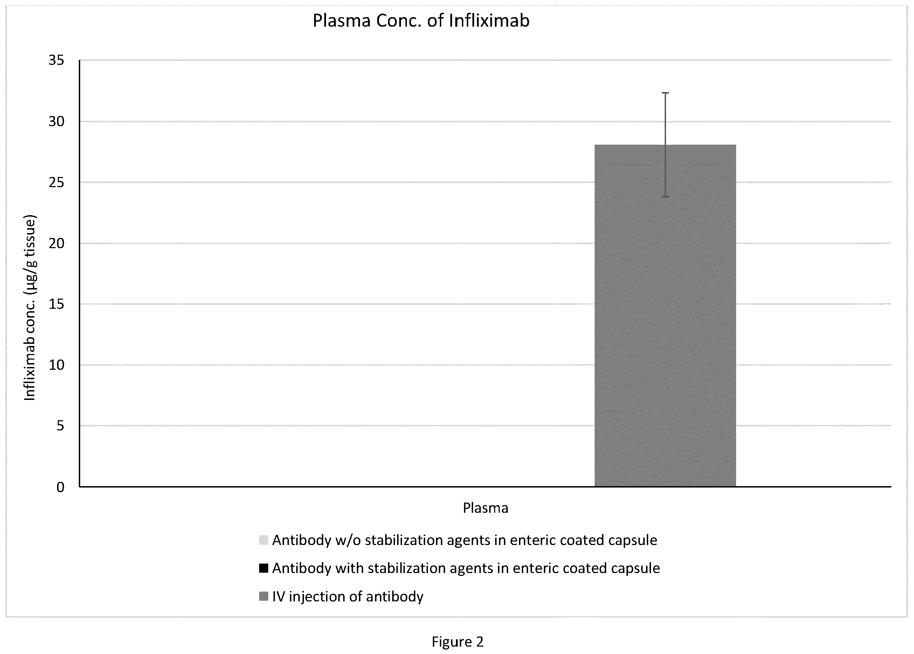

The results are shown in the figures 1 and 2.

The results demonstrated superior antibody concentration in the intestinal tissue (ileo-colonic and colonic) after formulating with stabilization agents in enteric coated capsule, compared to formulating without stabilization agents and IV injection of the same dose of antibody. No plasma exposure of antibody was observed after capsule dosing (with and without stabilization agents), while the IV injection dose showed high concentration of antibody floating in the systemic circulation.

## Claims

1. A pharmaceutical composition which comprises
(i) as active ingredient an antibody or an antigen binding fragment thereof,
(ii) carnosine and/or diglycine and
(iii) optionally aprotinin, or a fragment, or an analog thereof having at least 70% similarity to SEQ ID NO:1, wherein said analog or fragment of aprotinin acts as an enzyme inhibitor;
and wherein the antibody is stabilized in the presence of intestinal fluid by reducing degradation of the antibody in the ileum and/or colon.

2. A pharmaceutical composition as claimed in claim 1 in a liquid, solid or semi-solid form suitable for oral or rectal administration.

3. A pharmaceutical composition as claimed in claim 2, in a solid form suitable for oral administration, said composition having an enteric coating, preferably adapted for selective release of the protein in the lower gastro-intestinal tract.

4. A pharmaceutical composition as claimed in any one of the preceding claims, wherein said pharmaceutical composition is in a solid dosage form coated with a mixture of a digestible polysaccharide and a film-forming material which has a solubility threshold at pH 6.0 or above.

5. A pharmaceutical composition as claimed in claim 4, wherein the digestible polysaccharide is selected from the group consisting of starch; amylose; amylopectin; chitosan; chondroitin sulfate; cyclodextrin; dextran; pullulan; carrageenan; scleroglucan; chitin; curdulan and levan;

6. A pharmaceutical composition as claimed in claims 4 and 5, in which the film-forming material is an acrylate polymer, a cellulose polymer or a polyvinyl-based polymer, preferably cellulose acetate phthalate; cellulose acetate trimellitate; hydropropylmethylcellulose acetate succinate; and polyvinyl acetate phthalate.

7. A pharmaceutical composition as claimed in any one of the preceding claims suitable for oral administration.

8. A pharmaceutical composition as claimed in 7 which is a solid dosage form comprising a core comprising as active ingredient an antibody or fragment thereof, carnosine and/or diglycine and optionally aprotinin, or a fragment, or an analogue thereof, and a delayed release coating for the core.

9. A pharmaceutical composition as claimed in claim 1 or claim 2 suitable for rectal administration

10. The pharmaceutical composition as claimed in claim 9 wherein the composition is an enema formulation.

11. A composition as claimed in any one of claims 1 to 10 for use in a method of treating or preventing a disease or condition in a subject which comprises administering the composition to the subject via the ileum and/or the colon wherein the disease or condition is inflammatory bowel disease; irritable bowel syndrome; constipation; diarrhoea; infection; autoimmune disease or cancer.

12. A composition as claimed in any one of claims 1 to 10 for use in therapy.

13. The use of carnosine and/or diglycine for the stabilisation of an antibody or antigen binding fragment thereof in a pharmaceutical composition as defined in any one of claims 1 to 10, wherein the stabilisation results in reduced degradation of the antibody or fragment thereof once the composition is administered to the ileum and/or colon.

14. The use according to claim 13, further comprising the use of aprotinin or a fragment, or an analog thereof having at least 70% similarity to SEQ ID No.1, wherein said analog or fragment of aprotinin enhances the stability of the antibody or antigen binding fragment thereof.

## Patentansprüche

1. Eine pharmazeutische Zusammensetzung, umfassend
(i) als Wirkstoff einen Antikörper oder ein Antigenbindendes Fragment davon,
(ÎÎ) Carnosin und/oder Diglycin und
(ÎÎÎ) optional Aprotinin oder ein Fragment oder ein Analogon davon mit mindestens 70% Ähnlichkeit mit SEQ ID NO:1,
wobei das Analogon oder Fragment von Aprotinin als Enzymhemmer agiert;
und wobei der Antikörper bei Vorhandensein von Darmflüssigkeit durch Reduzierung des Abbaus des Antikörpers im Ileum und/oder Dickdarm stabilisiert wird.

2. Pharmazeutische Zusammensetzung nach Anspruch 1 in einer Flüssigkeit, einer festen oder halbfesten Form, die für eine orale oder rektale Verabreichung geeignet ist.

3. Pharmazeutische Zusammensetzung nach Anspruch 2, in einer festen Form, die für eine orale Verabreichung geeignet ist, wobei diese Zusammensetzung eine Magensaftresistenz hat, vorzugsweise angepasst an eine selektive Freigabe des Proteins im unteren Magen-Darm-Trakt.

4. Pharmazeutische Zusammensetzung nach einem der vorstehenden Ansprüche, wobei diese pharmazeutische Zusammensetzung in einer festen Dosierungsform vorliegt, beschichtet mit einer Mischung aus verdaulichem Polysaccharid und einem filmbildenden Material, das eine Löslichkeitsschwelle von pH 6,0 oder höher aufweist.

5. Pharmazeutische Zusammensetzung nach Anspruch 4, wobei das verdauliche Polysaccharid ausgewählt ist aus der Gruppe bestehend aus Stärke; Amylose; Amylopektin; Chitosan; Chondroitinsulfat; Cyclodextrin; Dextran; Pullulan; Carrageenan; Skleroglucan; Chitin; Curdulan und Levan;

6. Pharmazeutische Zusammensetzung nach Anspruch 4 und 5, bei denen das filmbildende Material ein Acrylatpolymer, ein Zellulosepolymer oder ein Polyvinyl-basiertes Polymer ist, vorzugsweise Celluloseacetatphthalat; Celluloseacetattrimellitat; Hydropropylmethylcelluloseacetatsuccinat und Polyvinylacetatphthalat.

7. Pharmazeutische Zusammensetzung nach einem der vorstehenden Ansprüche, die für eine orale Verabreichung geeignet ist.

8. Pharmazeutische Zusammensetzung nach Anspruch 7, in einer festen Dosierungsform, umfassend einen Kern, der als Wirkstoff einen Antikörper oder ein Fragment davon umfasst, Carnosin und/oder Diglycin und optional Aprotinin oder ein Fragment oder ein Analogon davon und eine verzögerte Freigabebeschichtung für den Kern.

9. Pharmazeutische Zusammensetzung nach Anspruch 1 oder Anspruch 2, die für eine rektale Verabreichung geeignet ist.

10. Pharmazeutische Zusammensetzung nach Anspruch 9, wobei die Zusammensetzung eine Einlaufformulierung ist.

11. Zusammensetzung nach einem der Ansprüche 1 bis 10 für die Verwendung in einem Verfahren zur Behandlung oder Prävention einer Krankheit oder eines Zustands in einem Patienten, das das Verabreichen der Zusammensetzung an den Patienten über das Ileum und/oder den Dickdarm umfasst, wobei die Krankheit oder der Zustand eine entzündliche Darmerkrankung; Reizdarmsyndrom; Verstopfung; Durchfall; Infektion; Autoimmunerkrankung oder Krebs ist.

12. Zusammensetzung nach einem der Ansprüche 1 bis 10 für die Verwendung in einer Therapie.

13. Verwendung von Carnosin und/oder Diglycin zur Stabilisierung eines Antikörpers oder eines antigenbindenden Fragments davon in einer pharmazeutischen Zusammensetzung wie in einem der Ansprüche 1 bis 10 definiert, wobei die Stabilisierung zu einem reduzierten Abbau des Antikörpers oder Fragments davon führt, sobald die Zusammensetzung dem Ileum und/oder Dickdarm verabreicht wird.

14. Verwendung nach Anspruch 13, ferner umfassend die Verwendung von Aprotinin oder einem Fragment oder einem Analogon davon mit mindestens 70% Ähnlichkeit zu SEQ ID No.1, wobei das Analogon oder Fragment von Aprotinin die Stabilität des Antikörpers oder antigenbindenden Fragments davon stärkt.

## Revendications

1. Composition pharmaceutique qui comprend
(i) comme ingrédient actif, un anticorps ou un de ses fragments se liant à l'antigène,
(ii) de la carnosine et/ou de la diglycine et
(iii) facultativement de l'aprotinine, ou un de ses fragments ou un de ses analogues ayant au moins 70 % de similarité avec la SEQ ID N° 1,
dans laquelle ledit analogue ou fragment d'aprotinine agit comme un inhibiteur d'enzyme ;
et dans laquelle l'anticorps est stabilisé en présence de liquide intestinal en réduisant la dégradation de l'anticorps dans l'iléon et/ou le côlon.

2. Composition pharmaceutique selon la revendication 1 sous forme liquide, solide ou semi-solide, appropriée pour une administration par voie orale ou rectale.

3. Composition pharmaceutique selon la revendication 2, sous une forme solide appropriée à l'administration par voie orale, ladite composition ayant un enrobage entérique, de préférence adapté à la libération sélective de la protéine dans le tractus gastro-intestinal inférieur.

4. Composition pharmaceutique selon l'une quelconque des revendications précédentes, ladite composition pharmaceutique se présentant sous une forme galénique solide enrobée d'un mélange d'un polysaccharide digestible et d'un matériau filmogène qui a un seuil de solubilité à pH 6,0 ou supérieur.

5. Composition pharmaceutique selon la revendication 4, dans laquelle le polysaccharide digestible est choisi dans le groupe consistant en amidon ; amylose ; amylopectine ; chitosane ; sulfate de chondroïtine ; cyclodextrine ; dextrane ; pullulane ; carraghénane ; scléroglucane ; chitine ; curdulane et lévane.

6. Composition pharmaceutique selon les revendications 4 et 5, dans laquelle le matériau filmogène est un polymère d'acrylate, un polymère de cellulose ou un polymère à base de polyvinyle, de préférence l'acétophtalate de cellulose ; le trimellitate d'acétate de cellulose ; l'acétosuccinate d'hydropropylméthylcellulose ; et l'acétophtalate de polyvinyle.

7. Composition pharmaceutique selon l'une quelconque des revendications précédentes, appropriée pour une administration par voie orale.

8. Composition pharmaceutique selon la revendication 7, qui est une forme galénique solide comprenant un noyau comprenant, comme ingrédient actif, un anticorps ou un de ses fragments, de la carnosine et/ou de la diglycine et facultativement de l'aprotinine, ou un de ses fragments ou un de ses analogues, et un enrobage à libération retardée destiné au noyau.

9. Composition pharmaceutique selon la revendication 1 ou la revendication 2, appropriée pour une administration par voie rectale.

10. Composition pharmaceutique selon la revendication 9, la composition étant une formulation de lavement.

11. Composition selon l'une quelconque des revendications 1 à 10 pour une utilisation dans un procédé de traitement ou de prévention d'une maladie ou d'un état chez un sujet, qui comprend l'administration de la composition au sujet par l'iléon et/ou le côlon, la maladie ou l'état étant une maladie inflammatoire de l'intestin ; le syndrome du côlon irritable ; la constipation ; la diarrhée ; une infection ; une maladie auto-immune ou un cancer.

12. Composition suivant l'une quelconque des revendications 1 à 10 pour une utilisation en thérapie.

13. Utilisation de carnosine et/ou de diglycine pour la stabilisation d'un anticorps ou d'un de ses fragments se liant à l'antigène dans une composition pharmaceutique selon l'une quelconque des revendications 1 à 10, dans laquelle la stabilisation a pour résultat une dégradation réduite de l'anticorps ou de son fragment une fois que la composition est administrée à l'iléon et/ou au côlon.

14. Utilisation selon la revendication 13, comprenant en outre l'utilisation d'aprotinine ou d'un de ses fragments ou d'un de ses analogues ayant au moins 70 % de similarité avec la SEQ ID N° 1, dans laquelle ledit analogue ou fragment d'aprotinine améliore la stabilité de l'anticorps ou de son fragment se liant à l'antigène,
